**Europäisches Patentamt**

**European Patent Office**

**Office européen des brevets**

(19)

(11) Publication number: **0 178 469**
**A2**

# (12) EUROPEAN PATENT APPLICATION

(21) Application number: **85111646.7**

(22) Date of filing: **14.09.85**

(51) Int. Cl.⁴: **A 61 B 17/12**

(30) Priority: **15.10.84 US 660757**
**07.01.85 US 689342**

(43) Date of publication of application:
**23.04.86 Bulletin 86/17**

(84) Designated Contracting States:
**AT BE CH DE FR GB IT LI NL SE**

(71) Applicant: **AMERICAN CYANAMID COMPANY**
**1937 West Main Street P.O. Box 60**
**Stamford Connecticut 06904(US)**

(72) Inventor: **Deniega, Jose C.**
**22 Farview Road**
**Brookfield Connecticut 06804(US)**

(74) Representative: **Wächtershäuser, Günter, Dr.**
**Tal 29**
**D-8000 München 2(DE)**

(54) **Nonmetallic surgical clip.**

(57) A nonmetallic surgical clip having two separate parts is described. The first part contains at least one arm. The second part contains at least means for approximating the arm. The clip is useful in ligating a blood vessel, e.g., an artery.

## NONMETALLIC SURGICAL CLIP

This invention relates to a nonmetallic surgical clip. More specifically, this invention relates to a nonmetallic surgical clip having two separate parts.

An embodiment is wherein each part of the clip contains an arm and means for approximating the cooperating surfaces of the two arms. An embodiment of the approximating means comprises a protrusion at the proximal end of one arm and an opening at the proximal end of the other arm. A specific embodiment is wherein the protrusion and the opening are in an oblique relationship to the arms.

The oblique relationship of the protrusion and the opening to the arms has at least two advantages. One advantage is that the height of the surgical clip decreases in an oblique relationship between the protrusion and the opening, and the arms, as compared to a perpendicular relationship. Also the greater the oblique relationship (that is, the further from a perpendicular or 90° relationship), the greater the decrease in the height of the surgical clip.

Another advantage is that the force required to open a surgical clip after the cooperating surfaces have been approximated increases with an oblique relationship between the protrusion and the opening, and the arms, as compared to a perpendicular relationship. Again, the greater the oblique relationship (that is, the further from a perpendicular or 90° relationship), the greater the increase in the force required to open a surgical clip.

Both of these advantages are extremely important, and may even be critical, in a surgical procedure, e.g. the ligating of a blood vessel. It is critical that a surgical ligating clip stop the flow of blood through a vessel. If the vessel ligated is an artery, then a variable force (due to the expansion and contraction of the heart muscle) will be acting on the clip. The surgical clip of this invention in an oblique relationship assures a wide margin of safety against even a partial opening of the approximated clip cooperating surfaces, after a blood vessel is ligated.

Also of extreme importance is the size of the surgical clip and mechanical applicator used in ligation. That is, surgical clips, although ideal in all properties except size, can be essentially without usefulness if, e.g., the vessel or tissue to be ligated is in a small area or if other vessels or organs impede the placement of the applicator and/or clip. Therefore, as a general statement, a better surgical clip is one that is smaller and/or thinner than the state of the art surgical clips. For all of the above reasons, the surgical clip of this invention is new, useful and unobvious over the known state of the art surgical clips.

Another embodiment is wherein the first part of the clip has two arms joined together at an apex. The improvement to the first part comprises a weakened portion and a bend in at least one arm. The bend is at the weakened portion. The weakened portion can be an opening in two (or more) tracks. The tracks are parallel to a length of a clip arm. The weakened portion can also be one or more perforations, indentations or deformations, which can be used either alone or with each other or with the opening in the tracks. It is to be understood that a weakened portion can be on each arm.

When on each arm, the weakened portions are relatively weaker than the cooperating surfaces of the surgical clip but rigid enough to maintain the shape of the clip. This combination of adequate rigidity of the clip in its noncrimped configuration and of the ability of the clip to preselectively begin to crimp at the weakened portions, enables the clip of this invention to ligate a tubular structure in the same manner as a metallic clip. That is, the clip of this invention can be placed around a blood vessel, and, when a force is applied to either or both nonparallel parts of the arms, the tips of the clip touch each other to form a diamond shaped enclosure around the tubular structure.

The advantages of the nonmetallic clip of this invention over the known prior art polymeric ligating clip are many. For example, because the clip forms a diamond shaped enclosure around (that is, it surrounds) the blood vessel, the possibility of the blood vessel extruding out during closing is essentially eliminated. Also, external (to the part of the clip that is in contact with the blood vessel) means for closing the clip can be used with this invention. External closing means eliminate the need to inspect and possibly clean or clear the blood vessel (or other tissue) before the clip cooperating surfaces can be approximated.

The nonmetallic material can be bioabsorbable. In one embodiment, the bioabsorbable material is a synthetic polymer. In another embodiment, the synthetic polymer has a glycolic acid ester linkage. In yet another embodiment, the polymer having a glycolic acid ester linkage is a homopolymer. In still yet another embodiment, the polymer having a glycolic acid ester linkage is a copolymer. As a specific embodiment, the copolymer has a glycolic acid ester linkage and a trimethylene carbonate linkage.

A nonmetallic surgical clip having two separate parts has been invented. The first part contains at least one arm. The second part contains at least means for approximating the arm.

One embodiment of this clip has two noncontiguous parts. Each part contains an arm and means for approximating the cooperating surfaces of the two arms.

In another embodiment, the first part has two arms joined together at an apex. The improvement to the first part is characterized by a weakened portion and a bend in at least one arm. The bend is at the weakened portion. The amount of the bend is such that at least a part of one arm is in an essentially parallel relationship with at least a part of the other arm.

## Description of the Drawings

Figure 1 is a side view of a surgical clip of this invention prior to the approximation of a blood vessel;

Figure 2 shows the surgical clip of this invention after approximation of the blood vessel shown in Figure 1;

Figure 3 is a bottom view of Figure 2;

Figures 4 and 5 are perspective views of Figures 1 and 2, respectively;

Figure 6 is a perspective view of a surgical clip of this invention showing a means for attaching one part of the surgical clip to a mechanical applicator;

Figure 7 is a side view of the surgical clip of Figure 6, after the clip cooperating surfaces have been approximated; and

Figure 8 is a front view of the mechanical applicator shown in Figures 6 and 7, taken along the plane 8-8 of Figure 7.

Figures 9 to 12 are side, top back and front views, respectively, of another surgical clip of this invention;

Figures 13 to 15 are side, top and front views, respectively, of a U-shaped member which is useful as a means for approximating the cooperating surfaces of the clip shown in Figures 9 to 12;

Figure 16 is a side view and Figure 19 is a perspective view showing the clip of Figures 9 to 12 and the U-shaped member of Figures 13 to 15 in a closed configuration;

Figure 17 is a perspective view showing a clip of Figure 9 as a first part, in an open configuration, and the U-shaped member of Figure 13 as a second part, both parts being contained by a mechanical applicator; and

Figure 18 is a perspective view of the clip of Figure 17 in a partially closed configuration, showing a diamond-shaped enclosure about a tubular vessel.

## DESCRIPTION OF THE INVENTION

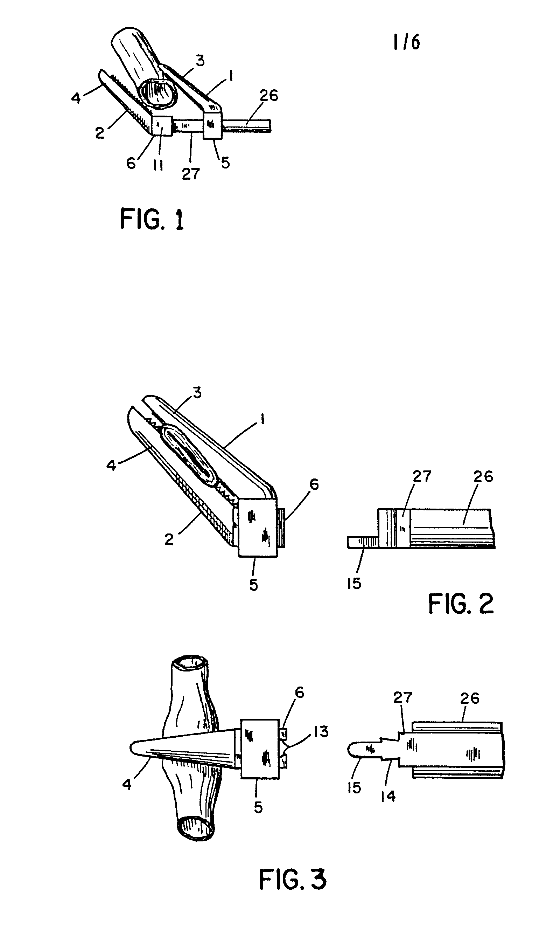

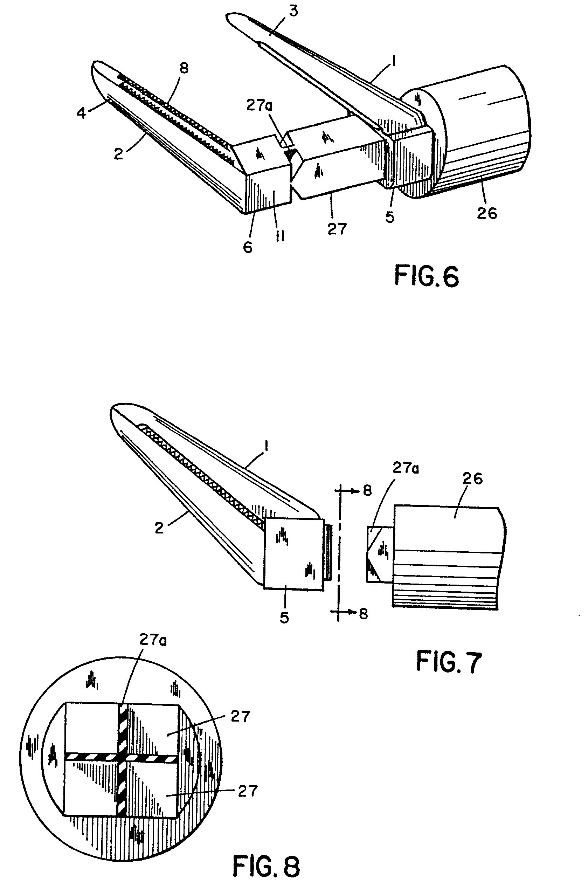

Figures 1 to 7 describe a surgical ligating clip having two separate and noncontiguous parts 1 and 2. Each part comprises an arm 3 and 4, respectively. Each part also comprises a means 5 and 6, respectively, for approximating the cooperating surfaces (see, e.g., element 8 in Figure 6) of the two arms 3 and 4. Referring to Figures 1 and 2, 4 and 5, or 6 and 7, the relationship of the approximating means 5 and 6 to the arms 3 and 4 is preferably an oblique relationship.

Referring to Figures 1, 4, and 6, the arms 3 and 4 are in an essentially parallel relationship. The cooperating surface of arm 3 can contain a raised portion. The cooperating surface 8 of arm 4 is at least partially serrated. The approximating means 6 can comprise a protrusion 11 at the proximal end of arm 4.

As implicitly shown in Figures 2, 5 and 7, the approximating means 5 can comprise an opening. The arms 3 and 4 are approximated by fitting the protrusion 11 into the opening.

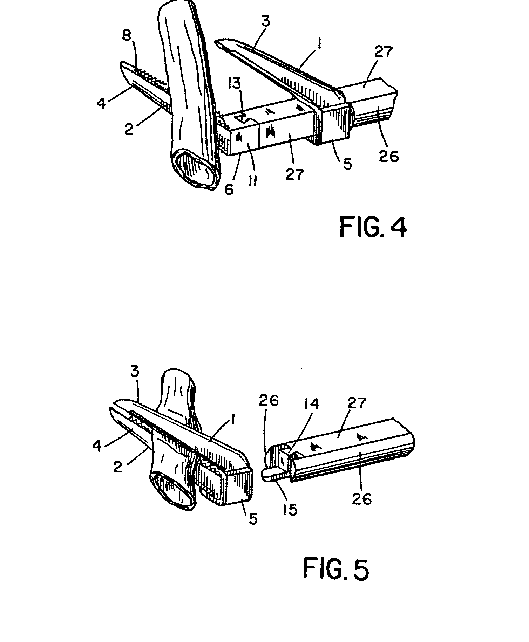

Referring to Figures 1 and 4, the protrusion 11 has a geometrical configuration essentially identical in shape but a cross-sectional perimeter slightly smaller than the inside perimeter of the approximating means 5 opening. Further, to assure a more permanent contact, the protrusion 11 can have a taper and the opening in the approximating means 5 can have an approximately equal reverse taper.

The protrusion 11 can be inserted, either manually or with a mechanical applicator, into the approximating means 5 opening. A mechanical applicator is preferred. Figures 1 to 5 and 6 to 8 show alternative distal ends of applicators which can be used to mechanically approximate the cooperating surfaces of arms 3 and 4.

In these figures, the surgical clip arms tend to be more than 90° away from the distal end of the mechanical applicators. However, it is to be understood that the surgical clip arms can be less than 90° away from the distal end of the mechanical applicators.

Referring to Figures 1 to 3 or 4 and 5, if a mechanical applicator is used, the approximating means 6 can contain a means for dovetailing the protrusion 11 onto the mechanical applicator. The dovetailing means can comprise a mortise 13 on the protrusion 11 and a tenon 14 on the mechanical applicator.

The approximating means 5 can be movably contained on the mechanical applicator. To accomplish this, the mechanical applicator can have a shaft 27. As shown, e.g., in Figure 4, the cross-sectional perimeter of the shaft 27 is essentially identical to the approximating means 5 opening. Also, the cross-sectional area of the shaft 27 is slightly less than the cross-sectional area of the approximating means 5 opening. The actual differences between these cross-sectional areas is not critical provided that a force applied to the approximating means 5 can overcome the static friction between the contact surfaces of the shaft 27 and the approximating means 5 opening.

The approximating means 6 can also contain a slot forward of and below the mortise 13. Referring to Figures 2, 3, and 5 the shaft 27 can contain a tab 15. The tab 15 is placed into the approximating means 6 slot during dovetailing of the protrusion 11 to the mechanical applicator. This prevents the ligating clip part 2 from disengaging from the applicator (e.g., as shown in Figures 1 and 4, during ligation of a tubular vessel).

Referring to Figures 6 to 8, a means for attaching the approximating means 6 to the shaft 27, and an alternative means for preventing disengagement of the ligating clip part 2 from a mechanical applicator is shown. The distal end of the shaft 27 is notched to obtain a weakened area 27a. Impliedly in this embodiment, the part 2 and the shaft 27 are manufactured from a sole piece of nonmetallic material, e.g. by machining or by injection molding. As shown in Figures 6 to 8, although the weakened portion 27a, is in the vertical, it is to be understood that the weakened portion can be any geometrical design, provided that the part 2 can be manually separated from the shaft 27 after the arms 3 and 4 are approximated.

The ligating clip of this invention can be mechanically applied to a living mammalian tissue, e.g. a tubular member such as a uterine artery. The application can be by a mechanical applicator. The type of mechanical applicator is not critical to the practice of this invention. That is, an applicator from the prior art can be used to apply the ligating clip to a living mammalian tissue. Examples of prior art applicators which can be used include, but are not limited to, a push-pull type, such as a plunger or piston device, and a fulcrum type, such as a medical device having scissors-like or pliers-like handles.

A push-pull applicator, such as a plunger can be used by pushing down on the plunger, for example, with the thumb. This causes the approximating means of part 1 to be engaged with the approximating means of the other part 2.

Referring to Figures 1 and 4 to 7, at the distal end of the plunger, part 2 of the clip is attached to a stationary member, for example interior rod 27. Part 1 of the clip is movably contained on the rod 27. The means for moving the clip part 1 (on the rod 27) can be an exterior rod 26. Referring to Figures 6 to 8, the exterior rod 26 can completely encircle the interior rod 27, or alternatively, referring to Figures 1 to 5, there can be a pair of exterior rods 26 (see specifically Figure 5). The pair of exterior rods 26 are positioned on opposite sides of the interior rod 27. The plunger can then be disengaged from the clip, for example by a latching means, or by a dovetailing means described above, after the cooperating surfaces of parts 1 and 2 have occluded a blood vessel or flattened a tubular member. Referring to Figures 2, 3 and 5, the force necessary to pull the tenon 14 from the mortise 13 is greater than the force necessary to occlude the largest blood vessel the clip can contain.

A fulcrum type applicator can be used, e.g., by bringing the two scissors-like handles together. This causes the approximating means of part 1 to be engaged with the approximating means of the other part 2. Referring to Figures 1 and 4 to 7, the distal end of the fulcrum type applicator can be essentially identical to that described above for the push-pull applicator.

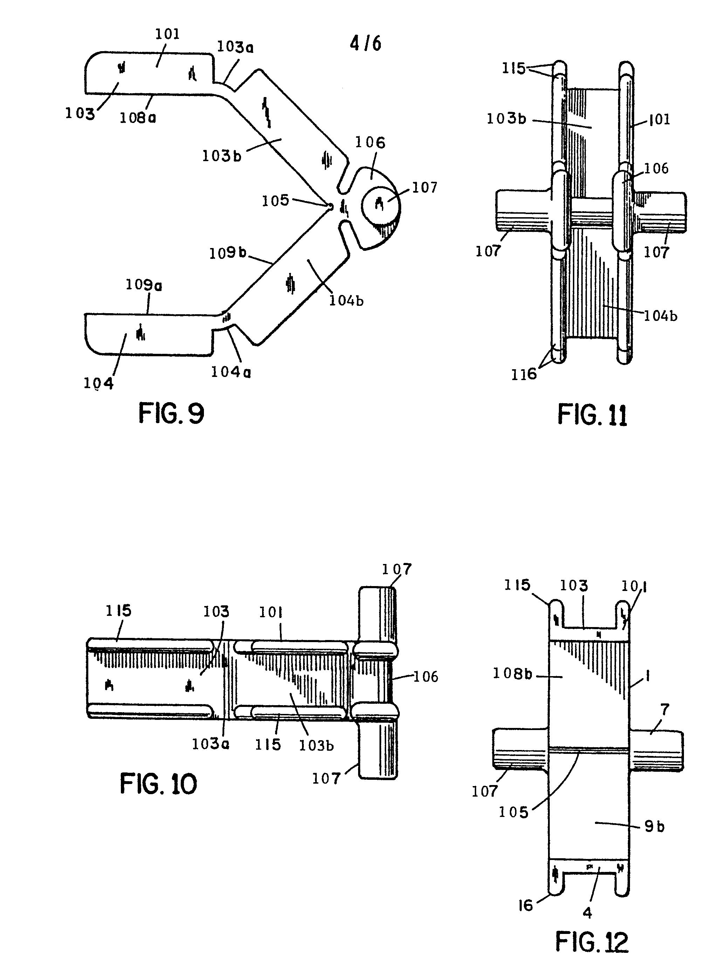

Figures 9 to 15 describe a surgical ligating clip having two separate parts 101 and 102, respectively.

Referring to Figures 9 to 12, part 101 comprises two essentially parallel arms 103 and 104. Preferably, the two arms 103 and 104 are essentially symmetrical. The two arms 103 and 104 are joined at their proximal ends to two nonparallel members 103b and 104b, respectively. The proximal ends of the two nonparallel members 103b and 104b are joined together at an apex 105.

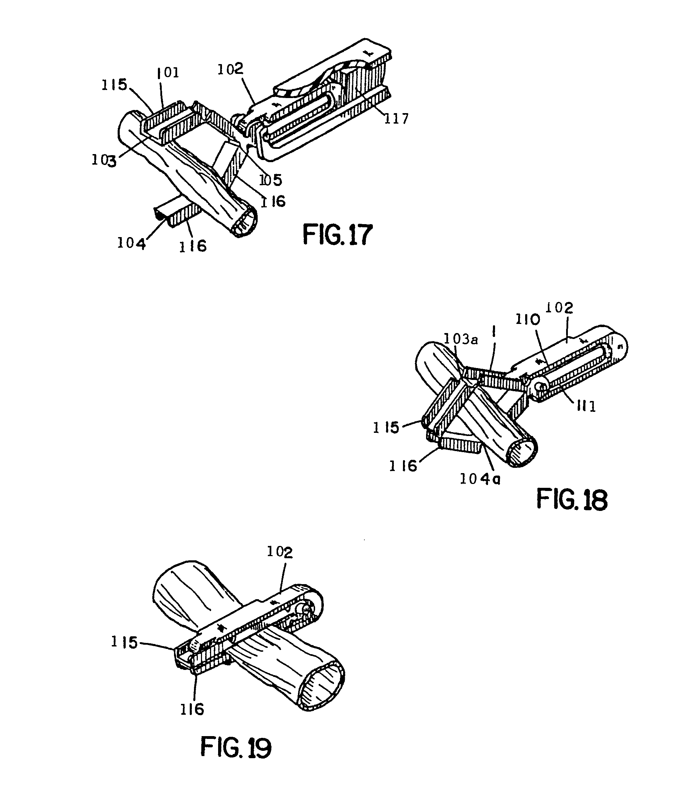

Preferably, part 101 also contains a protrusion 106. More preferably, the protrusion 106 is adjacent the apex 105. The protrusion 106 provides means for holding the part 101 in a contiguous relationship with the distal end of the part 102, as shown e.g. in Figure 17. Referring to Figure 17, the proximal ends of tracks 115 and 116 of part 101 can also be essentially contiguous with the distal end of part 102.

The arms 103 and 104 and the nonparallel members 103b and 104b are approximated by sliding the legs 110 and 111 of part 102 between the respective tracks 115 and 116 of part 101.

Referring to Figures 9 to 12 and 16 to 19, the arms 103 and 104 and the respective nonparallel members 103b and 104b are preferably in an essentially symmetrical relationship. Referring specifically to Figure 9, the cooperating surfaces 108a and 109a of arms 103 and 104 and/or 108b and 109b of members 103b and 104b can be flat. In another embodiment, either one or both of the cooperating surfaces 108a and 109a or 108b and 109b can be at least partially serrated. A final embodiment of either or both of the cooperating surfaces can be an elliptical or egg shaped configuration.

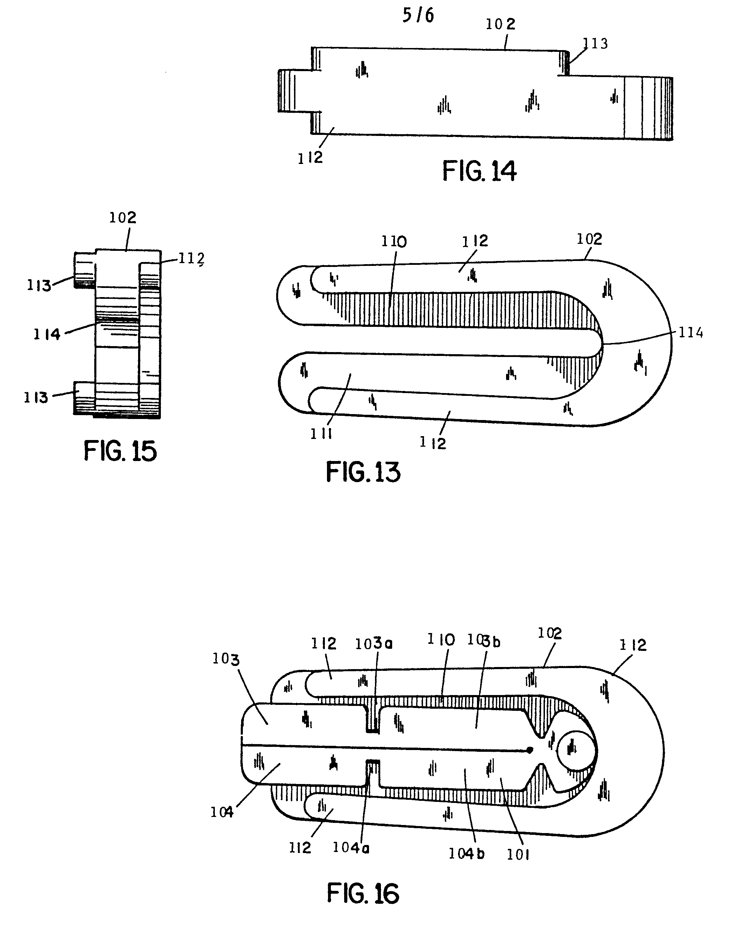

Referring to Figures 9, 13 to 16 and 18, part 102 comprises a means 110 and 111 for approximating the cooperating surfaces 108a and 109a of the two arms 103 and 104, and 108b and 109b of the two nonparallel members 103b and 104b. As specifically shown in Figures 13 to 16, part 102 comprises an essentially U-shaped member. In Figure 16 (see also Figure 17), each of the two legs 110 and 111 is essentially less than the length of the respective track 115 and 116. It is to be understood, however, that alternatively the two legs 110 and 111 can be essentially equal to the length of the respective tracks 115 and 116.

Reinforcing ribs 112 and 113 can be placed on either or both sides of the legs 110 and 111, and also (e.g., as shown with reinforcing rib 112) on the apex 114 of the U-shaped member. The reinforcing ribs tend to decrease, if not eliminate, the possible twisting motion of the legs 110 and 111 after the arms 103 and 104 and nonparallel members 103b and 104b are approximated, as shown in Figures 16 and 17.

Referring to Figures 9, 10, 16, 18 and 19, critical to the practice of this invention are the weakened portions 103a and 104a. In Figure 9, the weakened portions 103a and 104a are shown as openings in the tracks 115 and 116. However, it is to be understood that the weakened portions can be manufactured by other means, e.g. but not limited to, one or more perforations, or one or more continuous or discontinuous indentations or deformations. An example of two continuous indentations are shown in Figure 10 as double score lines adjacent to the weakened portion 103a. The perforation(s), indentation(s) or deformation(s) can be used either alone or in combination with each other, or in combination with the weakened portions 103a and 104a as shown in Figures 9 and 18.

To obtain the same configuration of part 101 shown in Figures 18 and 19, the perforation(s), indentation(s) or deformation(s) must be in essentially the same area as the weakened portions 103a and 104a.

It is to be understood that weakened portions 103a and 104a are relatively weaker than the cooperating surfaces 108a, 108b, 109a and 109b but rigid enough to maintain the shape of part 101 of the clip, e.g. as shown in Figure 17, until a force is applied by the U-shaped part 102. This combination of adequate rigidity of the clip part 101 in its noncrimped configuration and of the ability of the clip part 101 to preselectively begin to crimp at the weakened portions 103a and 104a enables the surgical ligating clip of this invention to ligate a tubular structure in the same manner as a metallic clip. That is, as shown in Figure 17, the clip is placed around the blood vessel. In Figure 18 the tips of part 101 touch each other to form a diamond shaped enclosure around the tubular structure, and in Figure 19 the arms 103 and 104 and the members 103b and 104b are approximated.

Referring again to Figures 9 to 13, the means 106 for holding the part 101 in a contiguous relationship with the distal end of the part 102 can further comprise one or more tabs 107. Either the protrusion 106 and/or the tabs 107 are movably contained by a mechanical applicator (see e.g., Figure 17). The means for containing the protrusion 106 and/or the tabs 107 at the distal end of the mechanical applicator are known in the prior art and can be, e.g., a notch at the distal end of the applicator. After the clip is in an approximated position, as shown e.g. in Figure 19, the protrusion 106 and/or the tabs 107 can separate from the notch, e.g. by a manual force transmitted to the distal end of the applicator.

The type of mechanical applicator useful in movably containing the ligating clip is not critical to the practice of this invention. That is, an applicator from the prior art can be used to apply the ligating clip to a living mammalian tissue, e.g. an artery. Examples of prior art applicators which can be used include, but are not limited to, a push-pull type, such as a plunger or piston device, and a fulcrum type, such as a medical device having scissors-like or pliers-like handles.

Referring to Figures 17 to 19, a push-pull applicator (such as a plunger device) can be used by pushing down on the plunger 117, for example with the thumb. This causes the legs 110 and 111 of part 102 to be engaged between the respective tracks 115 and 116 of the other part 101. Partially pushing down on the plunger effects the diamond-shape configuration of part 101 of the clip, as shown in Figure 18. Pushing down on the plunger for the complete stroke effects approximation of the tissue between the respective cooperating surfaces 108a and 109a, and 108b and 109b, as shown in Figure 19.

- 12 -

I CLAIM:

1. A nonmetallic surgical clip having two separate parts, the first part containing at least one arm, and the second part containing at least means for approximating the arm.

2. A clip of Claim 1 having two noncontiguous parts, each part containing an arm and means for approximating the cooperating surfaces of the two arms.

3. A clip of Claim 2 wherein the cooperating surfaces of said arms are in an essentially parallel relationship.

4. A clip of Claim 2 wherein said approximating means comprise a protrusion at the proximal end of one arm and an opening at the proximal end of the other arm.

5. A clip of Claim 4 wherein said protrusion and said opening are in an oblique relationship to said arms.

6. A clip of Claim 1, the first part having two arms joined together at an apex, the improvement to the first part characterized by a weakened portion and a bend in at least one arm, said bend being at said weakened portion and the amount of said bend being such that at least a part of one arm is in an essentially parallel relationship with at least a part of the other arm.

7. A clip of Claim 3 or 6 wherein at least a portion of one of the cooperating surfaces of said arms are serrated.

8. A clip of Claim 6 wherein said first part contains a protrusion, said protrusion adjacent said apex and opposite the proximal ends of said arms.

9. A clip of Claim 8 wherein said approximating means is a U-shaped member.

10. A clip of Claim 9 wherein the distal end of said U-shaped member is contacting said protrusion.

FIG. 1

FIG. 2

FIG. 3

FIG. 4

FIG. 5

FIG.6

FIG.7

FIG.8

0178469

4/6

FIG. 9

FIG. 11

FIG. 10

FIG. 12

5/6

FIG. 14

FIG. 15

FIG. 13

FIG. 16

FIG. 17

FIG. 18

FIG. 19